# EUROPEAN PATENT APPLICATION

(11) **EP 4 523 733 A1**
(43) Date of publication of application: **19.03.2025**
(21) Application number: 24801063.9
(22) Date of filing: 25.07.2024
(51) Int. Cl.: A61N 1/36, A61N 1/04

(54) **WIRELESS WEARABLE NEURAL STIMULATION DEVICE**

(30) Priority: 27.07.2023 KR 20230098449
(71) Applicant: Neurive Co., Ltd., Gimhae-si Gyeongsangnam-do 50969 (KR)
(72) Inventor: CHOI, Hyuk, Seoul 06095 (KR)
(74) Representative: Franke, Dirk
(86) International application number: PCT/KR2024/010832
(87) International publication number: WO 2025/023763

(57) **Abstract**

Proposed is a wireless wearable nerve stimulation apparatus configured to be worn on a user's ear. The stimulation apparatus includes an electrode part including at least one electrode configured to be brought into contact with the ear, a body part on which the electrode part is mounted and which is positioned on an outer side of the ear of the user in a wearing state of the stimulation apparatus, and a hanging part connected to the body part and configured to fix the stimulation apparatus by being hung on the helix of the ear of the user in the wearing state. The hanging part includes a first member positioned toward a front side of the helix and provided with a connection part that is connected to the body part, a second member which extends rearward from the first member and which includes a flexible material part that is configured such that at least a part thereof configured to be brought into contact with the ear of the user is formed of a flexible material, and a third member which extends rearward from the second member and which is positioned toward a rear side of the helix, the third member including a power source part for supplying power to the stimulation apparatus. As described above, since the stimulation apparatus is capable of being worn by being hung on the ear of the user, the wearing comfort is increased, so that the usability of the stimulation apparatus is increased. At this time, since the weight of the stimulation apparatus is distributed by dividing the hanging part for being hung on the ear of the user into three components and by positioning the power source part at the rear side of the helix of the ear of the user, there is an effect that the wearing comfort is increased.

## Description

### Technical Field

The present disclosure relates to an apparatus for stimulating a nerve. More particularly, the present disclosure relates to a nerve stimulation apparatus developed so as to facilitate wearing and using of the device for applying electrical stimulation and so on to a nerve positioned around the ear, the nerve including the vagus nerve.

### Background Art

The vagus nerve is one of the cranial nerves and is also called the tenth cranial nerve. The vagus nerve runs from the brain through the face and thorax to the abdomen. The vagus nerve is a mixed nerve that contains parasympathetic fibers and is involved in the regulation of parasympathetic nerves acting on the heart, lungs, and digestive tract. In the 12 pairs of cranial nerves, the vagus nerve has the longest and the most complex structure, and contains both sensory nerve fibers and motor nerve fibers.

It is known that a vagus nerve stimulation apparatus for the treatment of epilepsy and depression has been approved by the US Food and Drug Administration (FDA), and the vagus nerve stimulation apparatus targets the cervical branch of the vagus nerve located in the neck. However, this electrical stimulation of the cervical branch of the vagus nerve is a surgical procedure involving making an incision in the skin to expose the cervical branch of the vagus nerve, winding a coil, which is an electrical body, around the cervical branch, and grafting a microchip therein. Thus, it cannot be used in the field of self-treatment by the general public.

Meanwhile, the human ear is considered as a body part where electrical stimulation can be applied to the vagus nerve by externally applying electrical stimulation to the vagus nerve because the human ear is the main path through which the vagus nerve passes and is the region where the vagus nerve is located close to the skin surface. In oriental medicine, a method of treating diseases by ear acupuncture has been used. Since the ear is where the auricular branch of the vagus nerve is located, it can be said that the principle of ear acupuncture therapy is applied on the basis of stimulation of the vagus nerve. However, the use of acupuncture is problematic in that the use of acupuncture is not applicable to the general public.

Furthermore, considering that various nerves are positioned in the ear and positions around the ear, a technology for applying electrical stimulation by contacting an electrode to the ear or the position around the ear has been developed, but the technology has a disadvantage that the technology has a low usability due to discomfort in wearing and using the electrode.

### Disclosure

### Technical Problem

Accordingly, the present disclosure has been made keeping in mind the above problems occurring in the related art, and an objective of the present disclosure is to provide a nerve stimulation apparatus in which electrodes are disposed such that the apparatus is convenient to be worn and used and also stimulation of a nerve positioned in the ear is facilitated.

### Technical Solution

In order to achieve the objective described above, according to the present disclosure, there is provided a wireless wearable nerve stimulation apparatus configured to be worn on a user's ear, the stimulation apparatus including: an electrode part including at least one electrode configured to be brought into contact with the ear; a body part on which the electrode part is mounted and which is positioned on an outer side of the ear of the user in a wearing state of the stimulation apparatus; and a hanging part connected to the body part and configured to fix the stimulation apparatus by being hung on the helix of the ear of the user in the wearing state, wherein the hanging part includes: a first member positioned toward a front side of the helix and provided with a connection part that is connected to the body part; a second member which extends rearward from the first member and which includes a flexible material part that is configured such that at least a part thereof configured to be brought into contact with the ear of the user is formed of a flexible material; and a third member which extends rearward from the second member and which is positioned toward a rear side of the helix, the third member including a power source part for supplying power to the stimulation apparatus.

It is preferable that the third member is capable of being attached to and detached from the stimulation apparatus, wherein the third member and the second member are coupled to each other, and the second member is configured such that the second member is capable of being attached to and detached from the first member.

It is preferable that the connection part includes an adjustment rod capable of adjusting a distance between the body part and the first member, and the body part is configured such that the body part is capable of being rotated by using the adjustment rod as a rotation shaft.

It is preferable that the electrode part is capable of being attached to and detached from the body part, wherein the electrode part includes a first electrode configured to be brought into contact with the external acoustic meatus of the user, a second electrode configured to be brought into contact with a part of the helix of the ear of the user, and a base body to which the first electrode and the second electrode are connected, and the base body is capable of being attached to and detached from the body part.

At this time, the electrode part may be configured as a customized type electrode part in which an arrangement of the first electrode and an arrangement of the second electrode are adjusted according to a shape of the ear of the user.

In addition, a light-emitting apparatus may be mounted on the base body, at least a part of the base body may be a light-transmitting material, and the light-emitting apparatus may be mounted such that light is capable of being transmitted through the light-transmitting material.

A light-emitting apparatus may be mounted on the second member, the flexible material part may be a light-transmitting material, and the light-emitting apparatus may be mounted such that light is capable of being transmitted through the flexible material part.

The power source part may be a secondary battery.

### Advantageous Effects

In the present disclosure as described above, since the stimulation apparatus is capable of being worn by being hung on the ear of the user, the wearing comfort is increased, so that the usability of the stimulation apparatus is increased.

At this time, since the weight of the stimulation apparatus is distributed by dividing the hanging part for being hung on the ear of the user into three components and by positioning the power source part at the rear side of the helix of the ear of the user, there is an effect that the wearing comfort is increased.

In addition, since the third member of the hanging part where the power source part is positioned is capable of being replaced and used, using of the stimulation apparatus is capable of being realized by replacing only the part in which the power source part is contained. Therefore, the lifespan of the entire stimulation apparatus may extend, cleaning and disinfecting of the stimulation apparatus are capable of being easily performed, various power supply structures are capable of being applied by replacing the third member, and a biosensing module rather than a power supply structure is capable of being coupled to the third member. Since there is an effect that the usage shape and the design are capable of being changed by coupling a neckband, a headband, and so on, and various types of the biosensing modules having different sensing targets are capable of being replaced and used, the stimulation apparatus of the present disclosure may function as a type of platform for a biosensing apparatus.

Furthermore, since the electrode part in which the electrode is formed is configured such that the electrode part is capable of being attached to and detached from the body part, the management of the electrode is capable of being easily performed, and also there is an effect that the electrode part is capable of being replaced with a customized electrode part in which the position of the electrode is customized.

### Description of Drawings

FIG. 1 is a view illustrating a configuration of a wireless wearable nerve stimulation apparatus according to an embodiment of the present disclosure.
FIG. 2 is a view illustrating a coupling structure of an electrode part in the wireless wearable nerve stimulation apparatus according to an embodiment of the present disclosure.
FIG. 3 is a view illustrating a structure for attaching and detaching the electrode part to and from a body part in the wireless wearable nerve stimulation apparatus according to an embodiment of the present disclosure.
FIG. 4 is a view illustrating a structure for connecting the body part and a first member to each other in the wireless wearable nerve stimulation apparatus according to an embodiment of the present disclosure.
FIG. 5 is a view illustrating a structure for separating a power source part from the wireless wearable nerve stimulation apparatus according to an embodiment of the present disclosure.
FIG. 6 is a view illustrating a mounting structure of a second electrode in the wireless wearable nerve stimulation apparatus according to an embodiment of the present disclosure.

### Mode for Invention

Hereinafter, an embodiment of the present disclosure will be described in detail with reference to the accompanying drawings.

However, embodiments of the present disclosure may be modified in a variety of different forms, and the scope of the present disclosure is not limited to the embodiments described below. The shapes and sizes of the elements in the drawings may be exaggerated for clarity, and elements denoted by the same reference numerals in the drawings are the same elements.

Throughout the specification, it will be understood that when an element is referred to as being "connected" to another element, it can be directly connected to the other element or it can be electrically connected with the other element and intervening elements may be present therebetween. In addition, it will be further understood that when a part "comprises", "includes", or "has" an element, this means that other elements are not excluded but may be further included, unless otherwise stated.

Also, the terms such as "first", "second", etc. may be used to distinguish one element from another element, and the scope of the present disclosure must not be limited by these terms. For example, a first constitutive element may be referred as a second constitutive element, and the second constitutive element may be also referred to as the first constitutive element.

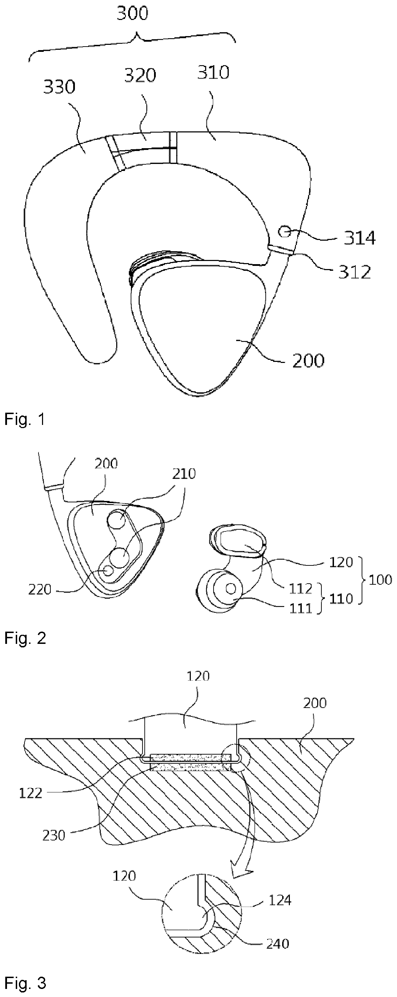

FIG. 1 is a view illustrating a configuration of a wireless wearable nerve stimulation apparatus according to an embodiment of the present disclosure, and FIG. 2 is a view illustrating a coupling structure of an electrode part in the wireless wearable nerve stimulation apparatus according to an embodiment of the present disclosure.

A wireless wearable nerve stimulation apparatus of the present disclosure includes an electrode part 100, a body part 200, and a hanging part 300.

The electrode part 100 includes at least one electrode 110 which is in contact with the user's ear and which is configured to apply electrical stimulation to the vagus nerve of the ear, and may preferably include at least two electrodes. Hereinafter, the description assumes a situation in which the vagus nerve that is a representative nerve among nerves located in the ear is stimulated. However, the stimulation is not limited to application on the vagus nerve only, and the stimulation is not only capable of being applied to the vagus nerve but also capable of being applied to nerves that pass through and around the ear.

In the human ear, the locations where the vagus nerve passes are the external acoustic meatus and the cymba concha. Here, the helicis crus that is a cartilage structure leading from the helix is located horizontally, and separates the cavum concha, which is the entrance to the external acoustic meatus, and the cymba concha.

In the present embodiment, a first electrode 111 configured to be brought into contact with the external acoustic meatus by being inserted into the external acoustic meatus and a second electrode 112 configured to be brought into contact with the helix are included. Specifically, the second electrode 112 is configured such that the second electrode 112 is brought into contact with the cymba concha located at a concave part in an upper side of the crus of the helix. At this time, the first electrode 111 inserted into the external acoustic meatus is configured to transmit sound through the external acoustic meatus. Although a sound generator of an earphone is capable of being formed in the electrode part, a structure in which the sound generator is mounted in the body part 200 and sound is capable of being transmitted through the electrode part 100 is applied.

In addition, in the present embodiment, the two electrodes are connected to a base body 120, and the base body 120 is configured such that the base body 120 is capable of being detached from and attached to the body part 200. As such, since the electrode part 100 is configured such that the electrode part 100 is capable of being detached from and attached to the body part 200, storing or managing the electrode part 100 by separating the electrode part 100 only is capable of being realized, and also replacing the electrode part 100 only is capable of being realized.

When only the electrode part 100 is separated and then is stored or managed, the electrodes that are configured to be brought into contact with the user's body are capable of being cleaned and disinfected so that the electrodes can be sanitized. The entire of the wireless wearable nerve stimulation apparatus of the present embodiment is configured to be hung and stored on a separated cradle, so that parts of the wireless wearable nerve stimulation apparatus configured to be brought into contact with the human body is capable of being disinfected by using an ultraviolet lamp and so on mounted on the cradle. At this time, in addition, since the stimulation apparatus of the present disclosure is configured such that the exclusive separation of the electrode part 100 is capable of being realized, the cleaning and disinfecting of the electrodes are capable of being more conveniently performed, so that there is an advantage that the stimulation apparatus of the present disclosure is capable of being more hygienically managed.

In addition, since the electrode part 100 equipped with the electrodes in which malfunctions frequently occur due to frequent contact with the human body during using the stimulation apparatus and which has a short lifespan is replaced and used, there is an advantage that there is no need to replace the entire of the stimulation apparatus due to malfunctions occurring in the electrodes or a durability issue. In addition, since the shape of the ear is different for each person, a customized electrode placement for each user may be required in a structure having a plurality of electrodes. Conventionally, a plurality of apparatuses is required to be provided for each user. However, in the present embodiment, since the electrode part 100 is capable of being replaced, there is an effect that one stimulation apparatus of the present disclosure is capable of being used to a plurality of users having the electrode parts including the customized electrode placement by changing the electrode part only.

FIG. 3 is a view illustrating a structure for attaching and detaching the electrode part to and from the body part in the wireless wearable nerve stimulation apparatus according to an embodiment of the present disclosure.

As a structure for attaching and detaching the electrode part 100 to and from the body part 200, various structures may be applied. In the present embodiment, a structure using a magnetic force of a magnet mounted on one or both of the electrode part 100 and the body part 200 is used. In the present embodiment, the electrode part 100 may be configured such that the electrode part 100 transmits electricity for electrical stimulation to the electrodes and sound is transmitted through the first electrode 111 inserted into the external acoustic meatus. To this end, the electrode part 100 is required to be accurately attached to a specific position of the body part 200 in which a terminal 210 for electricity transmission and a sound hole 220 for sound transmission are formed. When a magnetic force between a magnet 122 mounted in the base body 120 and a magnet 230 mounted in a position corresponding to the position of the magnet 122 is used, the user may conveniently align the electrode part 100 to an accurate position with a small force.

In addition, a mechanical coupling structure may be applied only or may be applied together with the attachment and detachment structure by the magnetic force. In the present embodiment, a fitting coupling structure is applied together, so that a coupling force between the electrode part 100 and the body part 200 is increased. Specifically, a recessed part 240 that is concave is formed on a coupling position of the body part 200, and a protrusion part 124 that corresponds to the recessed part 240 is formed on the base body 120 of the electrode part 100, so that the protrusion part 124 is inserted into the recessed part 240 when the electrode part 100 is coupled to the body part 200. At this time, in the embodiment illustrated, the size of the protrusion part 124 and the size of the recessed part 240 are adjusted such that the protrusion part 124 and the recessed part 240 are capable of being separated from each other when a predetermined amount of force is applied thereto. As another method, by using an elastic apparatus or an adjustment button, the degree to which the protrusion part 124 protrudes may be adjusted, so that the protrusion part 124 may not protrude in the attachment and detachment process and the protrusion part 124 may protrude only when the protrusion part 124 is in an attached state. The protrusion part 124 may be formed over an entire surface of the base body 120 or may be formed only on a part of the base body 120, and the recessed part 240 is formed such that the recessed part 240 corresponds to the protrusion part 124.

The body part 200 has a structure in which the electrode part 100 is capable of being detached from and attached to the body part 200. Furthermore, in a wearing state in which the user wears the stimulation apparatus of the present disclosure, the body part 200 is positioned outside the user's ear.

In the present embodiment, since the electrode part 100 is configured such that the electrode part 100 is capable of being detached from and attached to the body part 200, the electrode part 100 is capable of being used by replacing and changing the electrode part 100. Therefore, the weight and the manufacturing cost of the vagus nerve stimulation apparatus of the present disclosure are reduced by minimizing components included in the electrode part 100, and components for operating the vagus nerve stimulation apparatus of the present disclosure is mounted in the body part 200. For example, a driver, a stimulation generation apparatus, and so on for operating the vagus nerve stimulation apparatus may be mounted in the body part 200. Furthermore, in the driver, the stimulation generation apparatus, and so on for operating the vagus nerve stimulation apparatus, components for stimulating muscles and the vagus nerve or other nerves may be modified to the extent that characteristics of the present disclosure are not impaired or may be applied as it is.

In the present embodiment, the terminal 210 for transmitting electricity for electrical stimulation to the electrode part 100 that is detachable and the sound hole 220 for transmitting sound through the first electrode 111 configured to be inserted into the external acoustic meatus are formed in the body part 200, and the main function of the body part 200 is already described in the description of the configuration of the electrode part 100, so that the detailed description thereof will be omitted.

The hanging part 300 is a part configured to fix the stimulation apparatus of the present disclosure by being hung on the helix of the ear of the user in the wearing state, and includes a first member 310, a second member 320, and a third member 330.

The first member 310 is positioned on a front side of the helix in the wearing state, and is connected to the body part 200 via a connection part 312.

The connection part 312 is configured to connect the hanging part 300 that includes the first member 310 to the body part 200, and is also configured to perform a function for adjusting an arrangement shape of the hanging part 300 and the body part 200 according to a user.

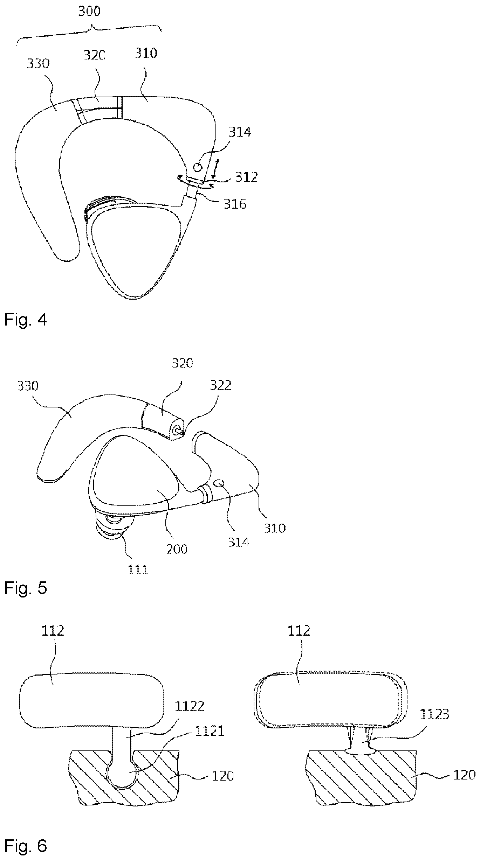

FIG. 4 is a view illustrating a structure for connecting the body part and the first member to each other in the wireless wearable nerve stimulation apparatus according to an embodiment of the present disclosure.

First, the connection part 312 includes an adjustment rod 316 that is capable of adjusting a distance between the first member 310 and the body part 200 by adjusting a length to which the adjustment rod 316 is exposed. The adjustment rod 316 is a structure in which all or part of the adjustment rod 316 is stored in one or both of the body part 200 and the first member 310, and the distance between the first member 310 and the body part 200 is capable of being adjusted by adjusting a length to which a stored part is exposed. Since people's ears vary in shape and size, a position of the body part 200 connected to the hanging part 300 may differ from person to person when the stimulation apparatus of the present disclosure is worn by hanging the hanging part 300 on the ear, and there is a problem that the electrodes of the electrode part 100 is difficult to be in contact with an accurate position when a distance between the body part 200 and the hanging part 300 is fixed. In the present disclosure, the distance between the first member 310 and the body part 200 is capable of being adjusted, so that various users are capable of using the stimulation apparatus of the present disclosure by adjusting the distance to fit their ear shape.

Furthermore, the body part 200 is configured such that the body part 200 is capable of being rotated by using the adjustment rod 316 as a rotation shaft, so that an angle of the body part 200 with respect to the first member 310 is capable of being adjusted. In addition to differences in the size and the shape of the ear, there are also differences in the angle of the helix and the external acoustic meatus. Therefore, in the present disclosure, the angle at which the first member 3100 and the body part 200 are disposed is capable of being adjusted, so that various users are capable of using the stimulation apparatus of the present disclosure by adjusting the angle to fit their ear shape.

When the position of the body part 200 is adjusted according to the ear shape of the user, the length and the angle of the adjustment rod 316 are capable of being freely adjusted. Furthermore, a fixing mechanism (not illustrated) configured to fix the length and the angle of the adjustment rod 316 after the adjustment is finished is provided, and the user is capable of releasing the fixing mechanism through an adjustment button 314 so as to perform the adjustment and then is capable of adjusting the fixing mechanism so that the fixing mechanism is operated.

Meanwhile, a component such as a wireless receiving apparatus may be mounted in the first member 310, thereby being capable of reducing the size of the body part 200. In addition, the size and the weight of the body part 200 may be reduced by distributing the components required for the operation of the vagus nerve stimulation apparatus to the first member 310 and the body part 200.

The second member 320 extends rearward from the first member 310, and is a part placed on the ear of the user in the wearing state.

Since the second member 320 is a part placed on the ear of the user and is a part where the weight of the vagus nerve stimulation apparatus is applied to the ear, so that at least a part in contact with the ear of the user is formed as a flexible material part having flexibility. Although the entire outer casing of the second member 320 in the present embodiment is formed of a flexible material, only lower side that is a part in contact with the ear of the user may be formed as a flexible material part formed of a flexible material as required.

Additional structures and features applied to the second member 320 will be described along with the third member 330.

The third member 330 extends rearward from the second member 320, and is a part positioned toward the rear side of the helix of the ear of the user in the wearing state.

The vagus nerve stimulation apparatus of the present disclosure uses electricity for electrical stimulation and so on, and power required for the vagus nerve stimulation apparatus is supplied through the third member 330. For example, a power supply line connected to an external power supply apparatus may be connected to the third member, or a power source part formed as a secondary battery may be contained inside the third member 330 as in the present embodiment. At this time, a power supply configuration such as a power supply line for transmitting power to the first member 310 and the body part 200 from the power source part mounted in the third member 330 is mounted in the second member 320.

In the present disclosure, the power supply line or the power source part for supplying power is mounted in the third member 330 positioned toward the rear side of the helix, so that the weight of the stimulation apparatus of the present disclosure in the wearing state is evenly distributed between a front side and a rear side by weight balancing the third member 330 with the body part 200 connected through the first member 310 positioned in front of the helix. In addition, when the power supply line is connected toward the rear side of the helix, inconvenience to the user may be minimized.

FIG. 5 is a view illustrating a structure for separating the power source part from the wireless wearable nerve stimulation apparatus according to an embodiment of the present disclosure.

The present disclosure has a characteristic in that the third member 330 in which the power source part is contained is capable of being separated from the stimulation apparatus of the present disclosure. At this time, since the secondary battery as the power source part configured to supply power to the vagus nerve stimulation apparatus is mounted in the third member 330, the third member 330 is capable of being separated from the stimulation apparatus of the present disclosure and then is capable of being charged separately or is capable of being replaced with the third member 330 that is additionally provided. Through this, the use time of the stimulation apparatus of the present disclosure may be increased. Furthermore, even when the power source part is required to be replaced due to a decrease in the lifespan of the secondary battery, only the part containing the power source part is capable of being replaced without replacing the entire stimulation apparatus.

A configuration capable of separating the third member 330 is not particularly limited, a configuration in which the second member 320 and the first member 310 are fixed and the third member 330 is configured to be detached from and attached to the second member is capable of being realized, or a configuration in which the third member 330 and the second member 320 are fixed and the second member 320 is configured to be detached from and attached to the first member 310 is capable of being realized. In the embodiment, the configuration in which the second member 320 is capable of being detached from and attached to the first member 310 is applied, and the second member 320 is capable of being coupled to and separated from the first member 310 through a connector 322 capable of transmitting power.

Meanwhile, by the structure in which the third member 330 is separated, it is also possible to apply third members having different shapes and functions. In the embodiment illustrated, the vagus nerve stimulation apparatus worn on both ears is in a form in which the vagus nerve stimulation apparatus is separated from each other, but the structure and the design of the stimulation apparatus is capable of being changed. That is, the structure of the stimulation apparatus may be modified such that the shape of the third member is manufactured as a neckband and the stimulation apparatus at the both sides is coupled to the single third member, or the shaped of the third member may be manufactured as a headband and the stimulation apparatus at the both sides is coupled to the single third member. In addition, the third member 330 may be replaced with the third member 330 that is newly developed or the advanced technical matters are applied therein. For example, the third member 330 may be configured such that a biosensing module is capable of being coupled to the third member. The biosensing module is a modularization of an apparatus provided with a biosensing function, and refers to a modularization of the apparatus to a structure capable of being replaced with or added to the third member of the present embodiment. Furthermore, other components except components essential for the biosensing function may use other components included in the nerve stimulation apparatus of the embodiment. Since biosensing equipment is required to be stably attached to the human body while the biosensing equipment is capable of performing a sensing function, various types of biosensing apparatuses that target different sensing areas are currently sold individually with configurations for attachment to the human body. This increases the manufacturing cost of the biosensing equipment, reduces the purchasing desire of the user, and makes the usage of the biosensing equipment inconvenient, thereby hindering the utility of the biosensing equipment. Since the nerve stimulation apparatus of the present embodiment is a wireless-type wearable apparatus, the nerve stimulation apparatus is capable of being stably attached to the human body while being in a state in which the sensing function is capable of being performed when a component capable of performing the biosensing function is added or replaced. Furthermore, through the modularization of the biosensing apparatus, various types of biosensing modules having different sensing targets are capable of being replaced and used, so that there is an effect that utilization of the biosensing module is highly increased. As such, the nerve stimulation apparatus of the present embodiment may function as a type of platform for the biosensing apparatus capable of being used by replacing various biosensing modules.

Furthermore, the wireless wearable nerve stimulation apparatus of the present disclosure may include various display parts capable of indicating and informing the user of the state of the stimulation apparatus by displaying light in the process of using and managing the stimulation apparatus, and a light-emitting apparatus for this purpose may be mounted in the stimulation apparatus.

First, the light-emitting apparatus may be mounted on the base body 120 of the electrode part 100 so that whether the electrode part 100 is fully coupled to the accurate position of the body part 200 is indicated, and a display part (not illustrated) in which a light-transmitting material that transmits light from the light-emitting apparatus is applied is positioned on the base body 120. An entire surface of the display part of the base body 120 exposed to the outside while the display part is attached to the body part 200 may be formed of the light-transmitting material, or the display part may be configured such that the light-transmitting material is exposed to only a part of the display part.

In addition, the light-emitting apparatus may also be mounted on the second member 320 so that a connection state of the power source part contained in the third member 330 and a remaining charge of the secondary battery is displayed. In the embodiment, a flexible material having light-transmitting characteristic is applied to the flexible material part, so that light from the light-emitting apparatus is capable of being transmitted through the flexible material part.

In addition, the light-emitting apparatus of the second member 320 and the light-emitting apparatus of the base body 120 may be respectively or simultaneously applied to indicate whether the stimulation apparatus is used, the usage state, a wireless connection state, and so on.

In the embodiment of the present disclosure described above, the position and the angle of the body part 200 are capable of being adjusted through the adjustment rod 316 applied to the connection part 312 that connects the body part 200 and the first member 310 to each other, so that the position of the electrode 110 is capable of being adjusted to a position suitable for applying electrical stimulation to the user. However, although it is easy to adjust the position of the first electrode 111 inserted into the external acoustic meatus by adjusting the position using the adjustment rod 316, the second electrode 112 configured to be brought into contact with the cymba concha may not be easily contact with the cymba concha by adjusting the adjustment rod 316 only according to the shape of the helix of the ear of the user and so on.

FIG. 6 is a view illustrating a mounting structure of the second electrode in the wireless wearable nerve stimulation apparatus according to an embodiment of the present disclosure.

In order to solve the problem described above, in the embodiment illustrated, a structure in which the position and the angle of the second electrode 112 are capable of being changed according to the shape of the helix of the ear of the user is applied.

First, the embodiment on the left illustrates a structure in which the second electrode 112 protrudes and is mounted in the base body 120, and a spherical joint 1121 and a connection rod 1122 are applied in the structure. As the structure in which the spherical joint 1121 is inserted into and positioned in the base body 120 and the second electrode 112 is positioned at an end of the connection rod 1122 that extends from the spherical joint 1121 is applied, the position of the second electrode 112 is capable of being changed according to a movement of the spherical joint 1121 due to a pressure applied to the second electrode 112.

Next, the embodiment on the right illustrates a structure in which the second electrode 112 protrudes and is mounted in the base body 120, and an elastic connection part 1123 is applied in the structure. The elastic connection part 1123 is a structure connecting the base body 120 and the second electrode 112 to each other, and is formed of an elastic material, so that the position of the second electrode 112 is capable of being changed as a shape of the elastic connection part 1123 is changed within a predetermined range according to a pressure applied to the second electrode 112.

In such structures, the second electrode 112 is capable of being completely in contact with the cymba concha of the user as the movement of the spherical joint 1121 and the change in shape of the elastic connection part 1123 occur according to the shape of the helix of the ear of the user.

As such, since the contact status of the first electrode 111 and/or the second electrode 112 may cause a problem, a configuration for checking the contact status of the electrode 110 may also be provided.

In order to check the contact status of the electrode 110, a touch sensor, a pressure sensor, a contact sensor, and so on may be applied. In addition a plurality of sensors may be mountined, when the number of sensors that are in contact with the skin of the user among the total sensors is determined to be less than a predetermined number, an alert may be generated to the user or the electrical stimulation may be stopped. At this time, in a sensor that can be applied through the electrode 110, such as a touch sensor or a contact sensor, a contact part where the electrode 110 is in contact with the skin may be divided into a plurality of contact parts and a plurality of sensors may be applied, respectively.

Furthermore, in order to improve and increase the contact characteristic of the electrode 110, a material in which an external appearance thereof is capable of being modified according to the skin surface of the user may be applied to a part where the electrode 110 is mounted. For example, by applying a material with greater flexibility in surface shape, such as a solid gel rather than a simple flexible material, the external appearance may adapt to a curved surface of the human body, thereby increasing the contact area of the electrode 110 with the skin.

Although the preferred embodiments of the present disclosure have been disclosed for illustrative purposes, those skilled in the art will appreciate that various modifications, additions and substitutions are possible, without departing from the technical idea of the present disclosure. Therefore, the scope of protection of the present disclosure should be determined by the scope of the appended claims, rather than the specific embodiments, and all technical ideas falling within the scope of the claims should be construed as being included in the scope of the present disclosure.

## Claims

1. A wireless wearable nerve stimulation apparatus configured to be worn on a user's ear, the stimulation apparatus comprising:
an electrode part comprising at least one electrode configured to be brought into contact with the ear;
a body part on which the electrode part is mounted and which is positioned on an outer side of the ear of the user in a wearing state of the stimulation apparatus; and
a hanging part connected to the body part and configured to fix the stimulation apparatus by being hung on the helix of the ear of the user in the wearing state,
wherein the hanging part comprises:
a first member positioned toward a front side of the helix and provided with a connection part that is connected to the body part;
a second member which extends rearward from the first member and which comprises a flexible material part that is configured such that at least a part thereof configured to be brought into contact with the ear of the user is formed of a flexible material; and
a third member which extends rearward from the second member and which is positioned toward a rear side of the helix, the third member comprising a power source part for supplying power to the stimulation apparatus.

2. The stimulation apparatus of claim **1,** wherein the third member is capable of being attached to and detached from the stimulation apparatus.

3. , The stimulation apparatus of claim **2,** wherein the third member and the second member are coupled to each other, and the second member is capable of being attached to and detached from the first member.

4. , The stimulation apparatus of claim **1,** wherein the connection part comprises an adjustment rod capable of adjusting a distance between the body part and the first member.

5. The stimulation apparatus of claim **4,** wherein the body part is capable of being rotated by using the adjustment rod as a rotation shaft.

6. The stimulation apparatus of claim **1,** wherein the electrode part is capable of being attached to and detached from the body part.

7. The stimulation apparatus of claim **6,** wherein the electrode part comprises a first electrode configured to be brought into contact with the external acoustic meatus of the user, a second electrode configured to be brought into contact with a part of the helix of the ear of the user, and a base body to which the first electrode and the second electrode are connected, and
the base body is capable of being attached to and detached from the body part.

8. The stimulation apparatus of claim 7, wherein the electrode part is configured as a customized type electrode part in which an arrangement of the first electrode and an arrangement of the second electrode are adjusted according to a shape of the ear of the user.

9. The stimulation apparatus of claim 7, wherein a light-emitting apparatus is mounted on the base body.

10. The stimulation apparatus of claim 9, wherein at least a part of the base body is a light-transmitting material, and the light-emitting apparatus is mounted such that light is capable of being transmitted through the light-transmitting material.

11. The stimulation apparatus of claim 1, wherein a light-emitting apparatus is mounted on the second member.

12. The stimulation apparatus of claim 11, wherein the flexible material part is a light-transmitting material, and the light-emitting apparatus is mounted such that light is capable of being transmitted through the flexible material part.

13. The stimulation apparatus of claim 1, wherein the power source part is a secondary battery.
